(19) Europäisches Patentamt
European Patent Office
Office européen des brevets

(11) EP 1 735 368 B1

(12) **EUROPEAN PATENT SPECIFICATION**

(45) Date of publication and mention of the grant of the patent:
**11.11.2009 Bulletin 2009/46**

(21) Application number: **05725254.6**

(22) Date of filing: **10.03.2005**

(51) Int Cl.:
***C08G 77/04*** *(2006.01)*

(86) International application number:
**PCT/US2005/007967**

(87) International publication number:
**WO 2005/090444 (29.09.2005 Gazette 2005/39)**

(54) **ALKYL-PHENYL SILSESQUIOXANE RESINS COMPOSITIONS**

ALKYL-PHENYL-SILSESQUIOXANHARZE ENTHALTENDE ZUSAMMENSETZUNGEN

COMPOSITIONS DE RESINES A BASE D'ALKYL-PHENYL SILSESQUIOXANE

(84) Designated Contracting States:
**AT BE BG CH CY CZ DE DK EE ES FI FR GB GR HU IE IS IT LI LT LU MC NL PL PT RO SE SI SK TR**

(30) Priority: **16.03.2004 US 553450 P**

(43) Date of publication of application:
**27.12.2006 Bulletin 2006/52**

(73) Proprietor: **Dow Corning Corporation**
**Midland, MI 48686-0994 (US)**

(72) Inventors:
- **HINTERMAN, Daniel, Michael**
  **Midland, MI 48642 (US)**
- **HORSTMAN, John, Bernard**
  **Midland, MI 48642 (US)**
- **STARK-KASLEY, Lori, Ann**
  **Midland, MI 48642 (US)**
- **WIEBER, Gary, Michael**
  **Midland, MI 48642 (US)**

(74) Representative: **Bailey, Lindsey**
**Elkington and Fife LLP**
**Thavies Inn House**
**3-4 Holborn Circus**
**London EC1N 2HA (GB)**

(56) References cited:
**EP-A- 0 812 870** **US-A- 5 173 290**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the invention

The present invention provides a cosmetic product that comprises a composition comprising a powder and an alkylphenyl silsesquioxane resin. The compositions can be incorporated into a variety of cosmetic products to enhance the durability and substantivity of powders after topical application.

### Background of the invention

Siloxane resins of the general formula $R_nSiO_{(4-n)/2}$, where R is an alkyl group and n is generally less than 1.8, are an important family of silicone polymers because of their utility in many commercial applications such as adhesive compositions and coatings applications. One particular subclass of siloxane resins, known as MQ resins (since they are comprised primarily of "M" units of the general formula $R_3SiO_{1/2}$ and "Q" units of the general formula $SiO_2$), have found utility in cosmetic formulations. In particular MQ resins are commonly used in "extended wear" or "transfer resistant" cosmetic formulations. In these formulations, MQ resins enhance the substantivity of the pigments or other formulation actives to skin after application creating a longer lasting, and hence extended wear product.

Representative examples of transfer resistant cosmetic compositions using MQ resins are found in US 6,071,503, US 6,074,654, US 6,139,823, US 6,340,466, WO 97/17058, and WO 97/17059 which disclose compositions comprising the combination of organosiloxane resins and fluid diorganosiloxane resins with a volatile carrier.

US 5,330,747 teaches cosmetics with enhanced durability using a film forming agent from a pressure sensitive adhesive composition comprising a trimethylsilyl endblocked resinous copolymer, a silanol endblocked polydiorganosiloxane fluid, and a phenyl containing polysiloxane fluid.

US 5,800,816 discloses cosmetic compositions having improved transfer resistance comprising: a) from about 0.1-60% by weight of trimethylated silica, b) from about 0.1-60% by weight of a volatile solvent having a viscosity of 0.5 to 100 centipoise at 25°C, c) 0.1-60% by weight of a nonvolatile oil having a viscosity of 200 to 1,000,000 centipoise at 25°C, d) 0.1-80% of a cosmetically acceptable carrier.

US 5,837,223 and US 6,036,947 teach transfer resistant high.luster cosmetic stick compositions comprising, by weight of the total composition: a) 10-70% of a volatile solvent having a viscosity of 0.5 to 20 centipoise at 25°C, b) 0.5-40% of a guerbet ester, and c) 0.1-20% of a siloxysilicate polymer.

GB 2,319,527 discloses fragrance releasing non-volatile polysiloxanes based on high molecular weight polydiorganosiloxane compounds where at least one or more of the organic substituents of the polymer is a radical derived from a fragrant alcohol.

Japanese examined patent publication 1994-72085 teaches makeup cosmetic compositions having improved water resistance and durability containing an organic silicone resin, a volatile silicone oil, and a make up powder.

In skin care formulations there is a need for improved siloxane resins that offer at least comparable extended wear and transfer resistance properties of the MQ resins presently used in cosmetic formulations, but having improved gloss (i.e. non-matte). Furthermore, there is a need for improving the solubility of phenyl silsesquioxane resins in commonly used personal care solvents.

The present inventors have discovered improved siloxane resins by combining alkyl ($R'SiO_{3/2}$) and phenyl ($C_6H_5SiO_{3/2}$) siloxy units. The resulting siloxane resins, herein referred to as alkyl-phenyl silsesquioxane resins, improve the substantivity of powders after topical application to skin. In particular, cosmetic formulations containing the present alkyl-phenyl silsesquioxane resins have improved durability over phenyl silsesquioxane resins alone.

### Summary of the invention

The present invention relates to a cosmetic product that comprises a composition comprising;

(A) a powder, and

(B) an alkyl-phenyl silsesquioxane resin comprising at least 60 mole % of siloxy units having the formula $(R'SiO_{3/2})_x(C_6H_5SiO_{3/2})_y$, where x and y have a value of 0.05 to 0.95, and R' is a monovalent hydrocarbon group having 2 to 8 carbon atoms.

The compositions can be incorporated into a variety of cosmetic products. The alkyl-phenyl silsesquioxane resins enhance the durability and substantivity of powders or pigments on skin after topical application from a formulation comprising the composition of the present invention.

Detailed description of the invention

The present invention relates to a cosmetic product that comprises a composition comprising;

(A) a powder, and
(B) an alkyl-phenyl silsesquioxane resin comprising at least 60 mole % of siloxy units having the formula $(R'SiO_{3/2})_x(C_6H_5SiO_{3/2})_y$, where x and y have a value of 0.05 to 0.95, and R' is a monovalent hydrocarbon group having 2 to 8 carbon atoms.

Component (A) is a powder, which is defined herein as a dry particulate matter having a particle size of 0.02-50 microns. The particulate matter may be colored or non-colored (for example white). Suitable powders include bismuth oxychloride, titanated mica, furned silica, spherical silica beads, polymethylmethacrylate beads, micronized teflon, boron nitride, acrylate polymers, aluminum silicate, aluminum starch octenylsuccinate, bentonite, calcium silicate, cellulose, chalk, corn starch, diatomaceous earth, fuller's earth, glyceryl starch, hectorite, hydrated silica, kaolin, magnesium aluminum silicate, magnesium carbonate, magnesium hydroxide, magnesium oxide, magnesium silicate, magnesium trisilicate, maltodextrin, montmorillonite, microcrystalline cellulose, rice starch, silica, talc, mica, titanium dioxide, zinc laurate, zinc myristate, zinc neodecanoate, zinc rosinate, zinc stearate, polyethylene, alumina, attapulgite, calcium carbonate, calcium silicate, dextran, kaolin, nylon, silica silylate, silk powder, serecite, soy flour, tin oxide, titanium hydroxide, trimagnesium phosphate, walnut shell powder, or mixtures thereof. The above mentioned powders may be surface treated with lecithin, amino acids, mineral oil, silicone oil, or various other agents either alone or in combination, which coat the powder surface and render the particles hydrophobic in nature.

The powder component (A) also comprises various organic and inorganic pigments. The organic pigments are generally various aromatic types including azo, indigoid, triphenylmethane, anthraquinone, and xanthine dyes which are designated as D&C and FD&C blues, browns, greens, oranges, reds, yellows, etc. Inorganic pigments generally consist of insoluble metallic salts of certified color additives, referred to as the Lakes or iron oxides. A pulverulent coloring agent, such as carbon black, chromium or iron oxides, ultramarines, manganese pyrophosphate, iron blue, and titanium dioxide, pearlescent agents, generally used as a mixture with colored pigments, or some organic dyes, generally used as a mixture with colored pigments and commonly used in the cosmetics industry, can be added to the composition. Pulverulent inorganic or organic fillers can also be added. These pulverulent fillers can be chosen from talc, micas, kaolin, zinc or titanium oxides, calcium or magnesium carbonates, silica, spherical titanium dioxide, glass or ceramic beads, metal soaps derived from carboxylic acids having 8-22 carbon atoms, non-expanded synthetic polymer powders, expanded powders and powders from natural organic compounds, such as cereal starches, which may or may not be crosslinked. Mention may be made in particular of talc, mica, silica, kaolin, nylon powders (in particular ORGASOL), polyethylene powders, Teflon, starch, boron nitride, copolymer microspheres such as EXPANCEL (Nobel Industrie), POLYTRAP, and silicone resin powder or microbeads (TOSPEARL from Toshiba, for example).

Component (B) is a alkyl-phenyl silsesquioxane resin comprising at least 60 mole % of siloxy units having the formula $(R'SiO_{3/2})_x(C_6H_5SiO_{3/2})_y$, where x and y have a value of 0.05 to 0.95, and R' is a monovalent hydrocarbon group having 2 to 8 carbon atoms. As used herein, x and y represent the mole fraction of $(R'SiO_{3/2})$ and $(C_6H_5SiO_{3/2})$ siloxy units (i.e. T-alkyl and T-phenyl siloxy units) relative to each other present in the alkyl-phenyl silsesquioxane resin. Thus, the mole fraction of $(R'SiO_{3/2})$ and $(C_6H_5SiO_{3/2})$ siloxy units each can independently vary from 0.05 to 0.95. However, the combination of $(R'SiO_{3/2})$ and $(C_6H_5SiO_{3/2})$ siloxy units present must total at least 60 mole %, alternatively 80 mole %, or alternatively 95 mole % of all siloxy units present in the alkyl-phenyl silsesquioxane resin.

R' can be a linear or branched alkyl such as ethyl, propyl, butyl, pentyl, hexyl, heptyl, or octyl group. Preferably, R' is propyl.

The alkyl-phenyl silsesquioxane resins can contain additional siloxy units such as (i) $(R^1{}_3SiO_{1/2})_a$ , (ii) $(R^2{}_2SiO_{2/2})_b$ , (iii) $(R^3SiO_{3/2})_c$ , or (iv) $(SiO_{4/2})_d$ units which are commonly known in the art, and also used herein, as M, D, T, and Q units respectively. The amount of each unit present in the alkyl-phenyl silsesquioxane resin can be expressed as a mole fraction of the total number of moles of all siloxy units present in the alkyl-phenyl silsesquioxane resin. Thus, the alkyl-phenyl silsesquioxane resin of the present invention comprise the units:

(i) $(R^1{}_3SiO_{1/2})_a$
(ii) $(R^2{}_2SiO_{2/2})_b$
(iii) $(R^3SiO_{3/2})_c$ ,
(iv) $(SiO_{4/2})_d$,
(v) $(R'SiO_{3/2})_x$ and
(vi) $(C_6H_5SiO_{3/2})_y$,

wherein

$R^1$, $R^2$, and $R^3$ are independently an alkyl group having from 1 to 8 carbon atoms, an aryl group, a carbinol group, or an amino group,
R' is a monovalent hydrocarbon having 2 - 8 carbon atoms,
a, b, c, and d have value of zero to 0.4,
x and y have a value of 0.05 to 0.95,
with the provisos that the value of x + y is equal to or greater than 0.60, and the value of a + b + c + d + x + y = 1

The $R^1$, $R^2$, and $R^3$ in the units of the alkyl-phenyl silsesquioxane resin are independently an alkyl group having from 1 to 8 carbon atoms, an aryl group, a carbinol group, or an amino group. The alkyl groups are illustrated by methyl, ethyl, propyl, butyl, pentyl, hexyl, and octyl. The aryl groups are illustrated by phenyl, naphthyl, benzyl, tolyl, xylyl, xenyl, methylphenyl, 2-phenylethyl, 2-phenyl-2-methylethyl, chlorophenyl, bromophenyl and fluorophenyl with the aryl group typically being phenyl.

For the purposes of this invention a "carbinol group" is defined as any group containing at least one carbon-bonded hydroxyl (COH) radical. Thus the carbinol groups may contain more than one COH radical such as for example

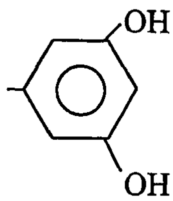

The carbinol group if free of aryl groups has at least 3 carbon atoms, or an aryl-containing carbinol group having at least 6 carbon atoms. The carbinol group free of aryl groups having at least 3 carbon atoms is illustrated by groups having the formula $R^4OH$ wherein $R^4$ is a divalent hydrocarbon radical having at least 3 carbon atoms or divalent hydrocarbonoxy radical having at least 3 carbon atoms. The group $R^4$ is illustrated by alkylene radicals such as $-(CH_2)_x-$ where x has a value of 3 to 10, $-CH_2CH(CH_3)-$, $-CH_2CH(CH_3)CH_2-$, $-CH_2CH_2CH(CH_2CH_3)CH_2CH_2CH_2-$, and $-OCH(CH_3)(CH_2)_x-$ wherein x has a value of 1 to 10.

The aryl-containing carbinol group having at least 6 carbon atoms is illustrated by groups having the formula $R^5OH$ wherein $R^5$ is an arylene radical such as $-(CH_2)_xC_6H_4-$ wherein x has a value of 0 to 10, $-CH_2CH(CH_3)(CH_2)_xC_6H_4-$ wherein x has a value of 0 to 10, $-(CH_2)_xC_6H_4(CH_2)_x-$ wherein x has a value of 1 to 10. The aryl-containing carbinol groups typically have from 6 to 14 atoms.

The amino group can be a primary, secondary, or tertiary amine. The amines are illustrated by groups having the formula $-R^6NH_2$ or $-R^6NHR^7NH_2$ wherein $R^6$ is a divalent hydrocarbon radical having at least 2 carbon atoms and $R^7$ is a divalent hydrocarbon radical having at least 2 carbon atoms. The group $R^6$ is typically an alkylene radical having from 2 to 20 carbon atoms. $R^6$ is illustrated by ethylene, propylene, $-CH_2CHCH_3-$, butylene, $-CH_2CH(CH_3)CH_2-$, pentamethylene, hexamethylene, 3-ethyl-hexamethylene, octamethylene, and decamethylene.

$R^7$ is typically an alkylene radical having from 2 to 20 carbon atoms. $R^7$ is illustrated by ethylene, propylene, $-CH_2CHCH_3-$, butylene, $-CH_2CH(CH_3)CH_2-$, pentamethylene, hexamethylene, 3-ethyl-hexamethylene, octamethylene, and decamethylene.

Typical amino groups are $-CH_2CH_2CH_2NH_2$ and

- $CH_2(CH_3)CHCH_2(H)NCH_3$, $-CH_2CH_2NHCH_2CH_2NH_2$, $-CH_2CH_2NH_2$,
- $CH_2CH_2NHCH_3$, $-CH_2CH_2CH_2CH_2NH_2$, $-(CH_2CH_2NH)_3H$, and
- $CH_2CH_2NHCH_2CH_2NHC_4H_9$.

Typically, $R^1$ is a methyl group, $R^2$ is a methyl or phenyl group, and $R^3$ is a methyl group.

Any individual D, T or Q siloxane units of the alkyl-phenyl silsesquioxane resins can also contain a hydroxy group and/or alkoxy group. Such siloxane units containing hydroxy and/or alkoxy groups are commonly found in siloxane resins having the general formula $R_nSiO_{(4-n)/2}$. The hydroxy groups in these siloxane resins typically result from the reaction of the hydrolyzable group on the siloxane unit with water. The alkoxy groups result from incomplete hydrolysis when alkoxysilane precursors are used or from exchange of alcohol with hydrolyzable groups. Typically, the weight percent of the total hydroxy groups present in the alkyl-phenyl silsesquioxane resin is up to 10%, alternatively, 5 %, or alternatively, 3%. Typically, the weight percent of the total alkoxy groups present in the alkyl-phenyl silsesquioxane resin is up to 20 %, alternatively up to 10%, or alternatively up to 5%.

The molecular weights of the alkyl-phenyl silsesquioxane resins are not restricted, but typically the number

average molecular weight ($M_N$) range from 500 to 10,000, or alternatively from 1,000 to 5,000.

The alkyl-phenyl silsesquioxane resins of the present invention can be prepared by any of the methods known in the art for preparing siloxane resins having the general formula $R_nSiO_{(4-n)/2}$ where R is an alkyl or aryl group and n is generally less than 1.8. Thus, the alkyl-phenyl silsesquioxane resins can be prepared by co-hydrolyzing the alkylsilane and a phenylsilane, each having three hydrolyzable groups such as a halogen or alkoxy group present in the silane molecule. For example, the alkyl-phenyl silsesquioxane resins can be obtained by co-hydrolyzing propyltrimethoxysilane, propyltriethoxysilane, or propyltripropoxysilane, with phenyltrimethoxysilane, phenyltriethoxysilane, or phenyltripropoxysilane. Alternatively, propyltrichlorosilane can be co-hydrolyzed with phenyltrichlorosilane to produce the alkyl-phenyl silsesquioxane resins of the present invention. Typically, the co-hydrolysis is performed in an alcohol or hydrocarbon solvent. Alcohols suitable for these purposes include methanol, ethanol, n-propyl alcohol, isopropyl alcohol, butanol, methoxy ethanol, ethoxy ethanol, or similar alcohols. Examples of hydrocarbon-type solvents which can also be concurrently used include toluene, xylene, or similar aromatic hydrocarbons; hexane, heptane, isooctane, or similar linear or partially branched saturated hydrocarbons; and cyclohexane, or similar aliphatic hydrocarbons.

The additional M, D, T, and Q units, as described supra, can be introduced into the alkyl-phenyl silsesquioxane resins by reacting an additional organosilane(s), selected to produce the desired siloxy unit in the resulting resin during the co-hydrolysis of the alkylsilane and phenylsilane. For example, reacting methoxytrimethylsilane, dimethoxydimethylsilane, trimethoxymethylsilane, tetramethoxysilane (or alternatively the corresponding ethoxy or chlorosilane of each) will respectively introduce a M, D, T, or Q unit into the alkyl-phenyl silsesquioxane resin. The amount of these additional silanes present in the co-hydrolysis reaction are selected to meet the mole fraction definitions, as described supra.

Alternatively, the alkyl-phenyl silsesquioxane resins can be prepared by the reacting an alkyl silsesquioxane and a phenyl silsesquioxane resin using any method in the art known to effect reaction of M, D, T, and Q siloxane units. For example, an alkyl silsesquioxane resin and a phenyl silsesquioxane resin can be reacted by a condensation reaction in the presence of a catalyst. Typically the starting resins are contained in an aromatic hydrocarbon or siloxane solvent. Suitable condensation reaction catalysts are base catalysts including metal hydroxides such as potassium hydroxide and sodium hydroxide; metal salts such as silanolates, carboxylates, and carbonates; ammonia; amines; and titanates such as tetrabutyl titanates; and combinations thereof. Typically, the reaction of siloxane resins is affected by heating the reaction mixture to temperatures ranging from 50 to 140°C, alternatively 100 to 140°C. The reaction can be conducted in a batch, semi-continuous, or continuous process.

The alkyl-phenyl silsesquioxane resins of this invention are illustrated by propyl-phenyl silsesquioxane resins comprising the units;

$(CH_3CH_2CH_2SiO_{3/2})_x$

$(C_6H_5SiO_{3/2})_y$

propyl-phenyl silsesquioxane resins comprising the units;

$(CH_3CH_2CH_2SiO_{3/2})_x$

$(C_6H_5SiO_{3/2})_y$

$((CH_3)_3SiO_{1/2})_a$ ,

propyl-phenyl silsesquioxane resins comprising the units;

$(CH_3CH_2CH_2SiO_{3/2})_x$

$(C_6H_5SiO_{3/2})_y$

$((CH_3)_2SiO_{2/2})_b$ ,

propyl-phenyl silsesquioxane resins comprising the units;

$(CH_3CH_2CH_2SiO_{3/2})_x$

$(C_6H_5SiO_{3/2})_y$

$((CH_3)SiO_{3/2})_c$ ,

propyl-phenyl silsesquioxane resins comprising the units;

$(CH_3CH_2CH_2SiO_{3/2})_x$

$(C_6H_5SiO_{3/2})_y$

$(SiO_{4/2})_d$

propyl-phenyl silsesquioxane resins comprising the units;

$(CH_3CH_2CH_2SiO_{3/2})_x$

$(C_6H_5SiO_{3/2})_y$

$((CH_3)_3SiO_{1/2})_a$ ,

$(SiO_{4/2})_d$

propyl-phenyl silsesquioxane resins comprising the units;

$(CH_3CH_2CH_2SiO_{3/2})_x$

$(C_6H_5SiO_{3/2})_y$

$((CH_3)_3SiO_{1/2})_a$ ,

$((CH_3)SiO_{3/2})_c$,

propyl-phenyl silsesquioxane resins comprising the units;

$(CH_3CH_2CH_2SiO_{3/2})_x$

$(C_6H_5SiO_{3/2})_y$

$((CH_3)_3SiO_{1/2})_a$ ,

$((CH_3)_2SiO_{2/2})_b$ ,

propyl-phenyl silsesquioxane resins comprising the units;

$(CH_3CH_2CH_2SiO_{3/2})_x$

$(C_6H_5SiO_{3/2})_y$

$((CH_3)_2SiO_{2/2})_b$ ,

$((CH_3)SiO_{3/2})_c$,

propyl-phenyl silsesquioxane resins comprising the units;

$(CH_3CH_2CH_2SiO_{3/2})_x$

$(C_6H_5SiO_{3/2})_y$

$((CH_3)_2SiO_{2/2})_b$ ,

$(SiO_{4/2})_d$

propyl-phenyl silsesquioxane resins comprising the units;

$(CH_3CH_2CH_2SiO_{3/2})_x$

$(C_6H_5SiO_{3/2})_y$

$((CH_3)SiO_{3/2})_c$,

$(SiO_{4/2})_d$

propyl-phenyl silsesquioxane resins comprising the units;

$(CH_3CH_2CH_2SiO_{3/2})_x$

$(C_6H_5SiO_{3/2})_y$

$((CH_3)_3SiO_{1/2})_a$ ,

$((CH_3)_2SiO_{2/2})_b$ ,

$((CH_3)SiO_{3/2})_c$, and

$(SiO_{4/2})_d$

propyl-phenyl silsesquioxane resins comprising the units;

$(CH_3CH_2CH_2SiO_{3/2})_x$

$(C_6H_5SiO_{3/2})_y$

$((CH_3)(H_2NCH_2CH_2CH_2SiO_{2/2})_b$ ,

propyl-phenyl silsesquioxane resins comprising the units;

$(CH_3CH_2CH_2SiO_{3/2})_x$

$(C_6H_5SiO_{3/2})_y$

$((CH_3)(C_6H_5)SiO_{2/2})_b$ ,

wherein a, b, c, and d have value of zero to 0.4, x and y have a value of 0.05 to 0.95,
with the provisos that the value of x + y is equal to or greater than 0.60, and the value of a + b + c + d + x + y = 1.

Optionally, the alkyl-phenyl silsesquioxane resin can be dissolved in component C), a solvent. A volatile siloxane or organic solvent can be selected as optional component C) for dissolving or dispersing the alkyl-phenyl silsesquioxane resin before mixing with (A) the powder. Any volatile siloxane or organic solvent can be selected providing component B) is dispersible or miscible with the selected solvent. The volatile siloxane solvent can be a cyclic polysiloxane, a linear polysiloxane, or mixtures thereof. Some representative volatile linear polysiloxanes are hexamethyldisiloxane, octamethyltrisiloxane, decamethyltetrasiloxane, tetradecamethylhexasiloxane, and hexadecamethylheptasiloxane. Some representative volatile cyclic polysiloxanes are hexamethylcyclotrisiloxane, octamethylcyclotetrasiloxane, decamethylcyclopentasiloxane, and dodecamethylcyclohexasiloxane. The organic solvent can be an ester, an alcohol such as methanol, ethanol, isopropanol, butanol, or n-propanol, a ketone such as acetone, methylethyl ketone, or methyl isobutyl ketone; an aromatic hydrocarbon such as benzene, toluene, or xylene; an aliphatic hydrocarbon such as heptane, hexane, or octane; a glycol ether such as propylene glycol methyl ether, dipropylene glycol methyl ether, propylene glycol n-butyl ether, propylene glycol n-propyl ether, or ethylene glycol n-butyl ether, an acetate, such as ethyl acetate or butyl acetate, a halogenated hydrocarbon such as dichloromethane, 1,1,1-trichloroethane or methylene chloride, chloroform, dimethyl sulfoxide, dimethyl formamide, acetonitrile, tetrahydrofuran, or an aliphatic hydrocarbon such as white spirits, mineral spirits, isododecane, heptane, hexane or naphtha. Typically, the solvent is decamethylcyclopentasiloxane or isododecane.

There are no special requirements or conditions needed for effecting the mixing of components A) and B). Thus, any method in the art known to effect mixing of such compositions can be used. Components A) and B) can be optionally contained in a solvent, as described supra as component C). The mixing can be conducted in a batch, semi-continuous, or continuous process.

The weight ratio of component A) to component B) (i.e. A/B) in the mixture can Vary from 99:1 to 1:99, alternatively 85:15 to 15:85.

The alkyl-phenyl silsesquioxane resins are useful in a variety of cosmetic compositions. Thus, they can be used in nail polishes, make-ups, color cosmetics, foundations, blushes, lipsticks, lip balms, eyeliners, mascaras, and powders. Furthermore, it is anticipated that the compositions of the present invention can be combined with various other components to prepare the personal care products described infra. These components include additional surfactants, moisturizers, pigments, sunscreens, fragrances, emollients, commonly used to formulate such personal care products.

The alkyl-phenyl silsesquioxane resins are particularly useful to enhance the durability and substantivity of pigments after topical application from cosmetic or make up formulations.

## EXAMPLES

The following examples are presented to further illustrate the compositions and methods of this invention, but are not to be construed as limiting the invention. All parts and percentages in the examples are on a weight basis and all measurements were obtained at about 23°C, unless indicated to the contrary.

The representative alkyl silsesquioxane resins of these examples are described using the M, D, T, and Q designation for the siloxy units present in the resin. The superscripts further describe the alkyl or phenyl substitute present on the siloxy unit. As used herein, Pr is $CH_3CH_2CH_2$-, Ph is $C_6H_5$-, and $NH_2$ is $-CH_2CH_2CH_2NH_2$. The subscripts describe the mole fraction of the siloxy unit in the resin. Thus, an alkyl-phenyl siloxane resin having a mole fraction of 0.50 for each siloxy unit is designated herein as $T^{Pr}_{0.50}$ $T^{Ph}_{0.50}$.

### Example 1

*$T^{Pr}{}_{0.50}$:$T^{Ph}{}_{0.50}$ siloxane resin made from methoxysilanes*

A 4-neck reaction flask was loaded with 991.50 g phenyltrimethoxysilane, 821.60 g propyltrimethoxysilane, and 0.52 g FC-24. The flask was equipped with an air driven stir blade, thermometer, and a Dean Stark trap with condenser. Then, 323.26 g of water was added within 5 seconds, and the mixture heated to remove the methanol formed. When 80% of the methanol was removed, 747.93 g of toluene was added to produce a 60wt % mixture. Heating was resumed to remove volatiles. After the majority of the methanol was removed, 162.15g of water were then added to further hydrolyze any remaining methoxy groups. The reaction mixture was heated to reflux (75-115°C) to remove methanol and water. After the resulting resin mixture cooled, 3.38 g of $CaCO_3$ were added to neutralize the FC-24 present, followed by 4 g of $MgSO_4$ to remove any trace amounts of water. The mixture was filtered to remove the salts and stripped on a rotary evaporator at an oil bath temperature of 150-155°C and 0.4 mm Hg (53.3 Pa). The resulting resin was a clear colorless solid at room temperature.

### Example 2

*$T^{Pr}{}_{0.70}$:$T^{Ph}{}_{0.30}$ resin made from chlorosilanes*

A 5 L 4-neck reaction flask was loaded with 2,136.52 g of deionized water and 214.00 g of 2-propanol and heated to 70°C with a heating mantle. A 2 L Erlenmeyer flask was loaded with 483.63 g of toluene, 291.70 g of phenyltrichlorosilane, $PhSiCl_3$, and 572.10 g of propyltrichlorosilane, $PrSiCl_3$. The reagents in the Erlenmeyer flask were then added to the reaction flask via an addition funnel while maintaining a temperature of 74 - 78°C during addition by the use of an ice water bath and varying the addition rate. After the addition was completed, the reaction mixture was cooled slowly. The heating mantle was applied to slow the cooling rate, but no heat was applied. The reaction products were transferred into a 4L separatory funnel at 50°C to remove the water phase. The material left in the separatory funnel was transferred into a 3 L 3 neck round bottom flask, and the remaining water was then removed via azeotrope. An azeotropic wash was then done using 50.78 g of deionized water and 21.61 g of 2-propanol. The water was again removed via azeotrope. The resulting mixture was analyzed for acid content which showed 148 ppm HCl based on solution. Volatiles were then removed from the resulting reaction mixture via a rotary evaporator at 125-130°C and 0.3mm Hg (40 Pa) for 1 hour. The resulting resin was dissolved into butyl acetate to produce a 75 wt% solids resin solution.

Example 3

*$T^{Pr}_{0.90}$:$T^{Ph}_{0.10}$ resin made from chlorosilanes*

This resin was prepared via the procedure of example 2 by adding a mixture of 182.90 g of toluene, 103.76g of $PhSiCl_3$, and 784.20g of $PrSiCl_3$ to a mixture of 2,278.82 g of deionized water and 229.10 g of 2-propanol in the reaction flask. The resulting resin was diluted to 75 wt% solids with butyl acetate.

These materials were characterized by NMR and GPC, evaluated for gloss and tack at 35% solids in volatile solvent on a Leneta chart, evaluated in a foundation (color cosmetic) for durability. NMR characterization can be found in Table 1 and application results can be found in Table 2.

Example 4

*$T^{Pr}_{0.45}$:$T^{Ph}_{0.45}$ $D^{NH2}_{0.05}$ $M_{0.05}$ siloxane resin made from methoxysilanes*

A 4-neck reaction flask was loaded with 446.15 g phenyltrimethoxysilane, 369.6 g, propyltrimethoxysilane, 47.85 g of $Me(EtO)_2Si(PrNH_2)$ and 376.38 g of xylenes. The flask was equipped with an air driven stir blade, thermometer, and a condenser. Then, 116.0 g of water was added, and the mixture heated to remove the alcohol formed. 44.0 g of $Me_3SiOEt$, 78.26 g of water and 10.55 g of 1.0 M KOH $_{(aq)}$ was added and water and alcohol was stripped off in a Dean Stark trap while heating at reflux. Part way through the stripping 10.77 g of 1.0 M HCl was added to neutralize the KOH. After the majority of the alcohol and water was removed the material was filtered to remove the salts and stripped on a rotary evaporator at an oil bath temperature of 155-160°C and 0.6 mm Hg (80 Pa) for 1 hour. The resulting resin was a clear colorless solid at room temperature.

Example 5

*$T^{Pr}_{0.30}$:$T^{Ph}_{0.70}$ resin*

This resin was prepared using a procedure similar to Example 2.

Example 6

*$T^{Pr}_{0.32}$:$T^{Ph}_{0.31}$ $D^{NH2}_{0.05}$ $M_{0.33}$*

This resin was prepared using a procedure similar to Example 4.

Table 1:NMR & GPC Characterization

| Example # | NMR Characterization | Wt% OR | wt%OH | Mn | Mw | Polydispersity - Mw/Mn |
|---|---|---|---|---|---|---|
| Comparative #1 | $T^{Pr}_{1.0}$ | | 7.0% | 3470 | 11400 | 3.3 |
| Comparative #2 | $T^{Ph}_{1.0}$ | | | 1440 | 2120 | 1.47 |
| Example 1 | $T^{Pr}_{0.492}T^{Ph}_{0.502}$ | 1.8% | 5.0% | 2077 | 5156 | 2.48 |
| Example 2 | $T^{Pr}_{0.692}T^{Ph}_{0.306}$ | 1.8% | 7.0% | 2068 | 5171 | 2.50 |
| Example 3 | $T^{Pr}_{0.897}T^{Ph}_{0.103}$ | 1.7% | 7.2% | 2256 | 6736 | 2.99 |
| Example 4 | $T^{Pr}_{0.46}$:$T^{Ph}_{0.45}$ $D^{NH2}_{0.05}$ $M_{0.03}$ | 0.3% | 0.1% | 2750 | 6200 | 2.3 |
| Example 5 | $T^{Pr}_{0.30}T^{Ph}_{0.70}$ | | | 1860 | 3250 | 1.75 |
| Example 6 | $T^{Pr}_{0.32}$:$T^{Ph}_{0.31}$ $D^{NH2}_{0.05}$ $M_{0.33}$ | 1.2% | 0.2% | 1270 | 1620 | 1.3 |

Table 2: Application Results

| Example # | 60° Gloss | Tack | Foundation Durability - ΔE (Change in color) |
|---|---|---|---|
| No Resin - negative control | 55 | | 9.2 |
| Comparative #1 | 78.4 | Very tacky | 11.0 |
| Comparative #2 | 93.7 | Not Tacky | 11.12 |
| Example 1 | 89.3 | Not Tacky | 9.5 |
| Example 2 | 86.3 | Slightly tacky | 14.61 |
| Example 3 | 80.8 | Very tacky | 17.3 |
| Example 4 | 90.1 | Not Tacky | 3.8 |
| Example 5 | 89.3 | Slightly Tacky | 5.87 |
| Example 6 | 67 | Not Tacky | 11.4 |

**Foundation Formulation**

Pigment Premix:

50 wt% DC 245 Fluid
13.16 wt% Carde AS Titanium dioxide (caprylyl silane treated)
11.41 wt% Carde AS Red Iron Oxide(caprylyl silane treated)
18.26 wt% Carde AS Yellow Iron Oxide(caprylyl silane treated)
7.17 wt% Carde AS Black Iron Oxide(caprylyl silane treated)

Procedure:

1) Place DC 245 fluid in Waring Blender
2) Add titanium dioxide and mix by pressing the pulse button for 2 seconds for 15 seconds total.
3) Add red iron oxide and mix the same as titanium dioxide
4) Continue with the other pigments
5) When all materials have been dispersed, mix on high and shred for 30 sec to grind the pigments
6) Place premix into a round glass jar and place on pail roller for 6 hours.

Phase A

20.50 wt% Pigment Premix
7.50 wt% DC 5225C
8 wt% of a 50% resin solids in solvent

Phase B

54.80 wt % DI Water

1.0 wt% NaCl

0.20 wt% Polysorbate 20

Procedure for Liquid Water in oil Foundation

1) Put pigment dispersion on roller for 1 hour.
2) Weigh out resin and solvent to make a 50% solids dilution. Use oven and wheel to mix
3) Combine ingredients in Phase A, mix until uniform using a dual blade, turbulent style mixing action.
4) Combine ingredients in Phase B in separate beaker, mix until uniform using a magnetic stirrer
5) Increase mixing speed of Phase A to 1376 rpm and very slowly add Phase B through an addition funnel. This addition should take 10 mins.

Continue mixing for an additional 10 min.

**Foundation Durability Method: Gardner Abrasion Tester**

1) Cut collagen into 3.5" x 3" pieces, place one on each of the 3" x 2.5" polycarbonate blocks and put in the humidity chamber overnight. This chamber must be at a constant 98% relative humidity level.
2) Remove collagen and block from chamber. Secure collagen to block with Scotch tape taking care not to place any tape on the top of the block's surface.
3) Add approximately 1 gram of foundation to the collagen, beading it across the top of the block.Using a #8 Meyer rod, coat the collagen with the foundation by placing the rod on the bead of foundation and spreading it downward to the bottom of the block. The final coating weight should be approximately 0.2 grams. This operation may need to be repeated to obtain the proper coating weight. Remove any material from the sides of the block.
4) Allow sample on collagen to dry. Drying times vary with different samples. Entire sample must be free from any wetness before testing. Measure color of sample on collagen for the initial baseline color using a spectrophometer or colorimeter. L*, a*, and b* designate the place of the colored object in a tri-dimensional space.
5) Place block with collagen face-up on the Gardner Abrasion Tester making sure that the block is in the tester. The soft side of Velcro is attached to the insult block to abrade or insult the foundation sample on the collagen. The insult block rubs back and forth across the foundation sample. One insult consists of one back and forth motion. Insult the foundation sample on the collagen 20 times. The machine can be stopped at certain intervals to measure the color.
6) After the foundation sample is insulted 20 times, the color is read as L*, a*, b* and the change in color, ΔE, is calculated (see equation below). The number of insults, coating weight, and repetitions can be changed to fit the needs of the material being tested. This is up to the discretion of the operator.

$$\Delta L^*,\ \Delta a^* \text{ and } \Delta b^* = \text{value after abrasion} - \text{value at initial baseline before abrasion.}$$

$$\Delta E = (\Delta L^2 + \Delta a^{*2} + \Delta b^{*2})^{1/2}$$

With larger ΔE's, more foundation was removed and therefore the foundation is less durable.

## Claims

**1.** A cosmetic product that comprises a composition comprising;

(A) a powder, and
(B) an alkyl-phenyl silsesquioxane resin comprising at least 60 mole % of siloxy units having the formula $(R'SiO_{3/2})_x(C_6H_5SiO_{3/2})_y$,

where x and y have a value of 0.05 to 0.95, and
R' is a monovalent hydrocarbon group having 2 to 8 carbon atoms.

**2.** The cosmetic product of claim 1 wherein the alkyl-phenyl silsesquioxane resin comprises the units:

(i) $(R^1{}_3SiO_{1/2})_a$
(ii) $(R^2{}_2SiO_{2/2})_b$
(iii) $(R^3SiO_{3/2})_c$ ,
(iv) $(SiO_{4/2})_d$,
(v) $(R'SiO_{3/2})_x$ and
(vi) $(C_6H_5SiO_{3/2})_y$,

wherein

$R^1$, $R^2$ , and $R^3$ are independently an alkyl group having from 1 to 8 carbon atoms, an aryl group, a carbinol group, or an amino group,
R' is a monovalent hydrocarbon group having 2 to 8 carbon atoms,
a, b, c, and d have value of zero to 0.4,
x and y have a value of 0.05 to 0.95,

with the provisos that the value of x + y is equal to or greater than 0.60, and the value of a + b + c + d + x + y = 1.

**3.** The cosmetic product of claim 1 or claim 2 where R' is a propyl group.

**4.** The cosmetic product of any one of claims 1 to 3 comprising:

a propyl-phenyl silsesquioxane resin comprising the units:

$(CH_3CH_2CH_2SiO_{3/2})_x$

$(C_6H_5SiO_{3/2})_y$;

a propyl-phenyl silsesquioxane resin comprising the units:

$(CH_3CH_2CH_2SiO_{3/2})_x$

$(C_6H_5SiO_{3/2})_y$

$((CH_3)_3SiO_{1/2})_a$ ;

a propyl-phenyl silsesquioxane resin comprising the units:

$(CH_3CH_2CH_2SiO_{3/2})_x$

$(C_6H_5SiO_{3/2})_y$

$((CH_3)_2SiO_{2/2})_b$ ;

a propyl-phenyl silsesquioxane resin comprising the units:

$(CH_3CH_2CH_2SiO_{3/2})_x$

$(C_6H_5SiO_{3/2})_y$

$((CH_3)SiO_{3/2})_c$ ;

a propyl-phenyl silsesquioxane resin comprising the units:

$(CH_3CH_2CH_2SiO_{3/2})_x$

$(C_6H_5SiO_{3/2})_y$

$(SiO_{4/2})_d$ ;

a propyl-phenyl silsesquioxane resin comprising the units:

$(CH_3CH_2CH_2SiO_{3/2})_x$

$(C_6H_5SiO_{3/2})_y$

$((CH_3)_3SiO_{1/2})_a$ ,

$(SiO_{4/2})_d$ ;

a propyl-phenyl silsesquioxane resin comprising the units:

$(CH_3CH_2CH_2SiO_{3/2})_x$

$(C_6H_5SiO_{3/2})_y$

$((CH_3)_3SiO_{1/2})_a$ ,

$((CH_3)SiO_{3/2})_c$ ;

a propyl-phenyl silsesquioxane resin comprising the units:

$(CH_3CH_2CH_2SiO_{3/2})_x$

$(C_6H_5SiO_{3/2})_y$

$((CH_3)_3SiO_{1/2})_a$ ,

$((CH_3)_2SiO_{2/2})_b$ ;

a propyl-phenyl silsesquioxane resin comprising the units:

$(CH_3CH_2CH_2SiO_{3/2})_x$

$(C_6H_5SiO_{3/2})_y$

$((CH_3)_2SiO_{2/2})_b$ ,

$((CH_3)SiO_{3/2})_c$ ;

a propyl-phenyl silsesquioxane resin comprising the units:

$(CH_3CH_2CH_2SiO_{3/2})_x$

$(C_6H_5SiO_{3/2})_y$

$((CH_3)_2SiO_{2/2})_b$ ,

$(SiO_{4/2})_d$ ;

a propyl-phenyl silsesquioxane resin comprising the units:

$(CH_3CH_2CH_2SiO_{3/2})_x$

$(C_6H_5SiO_{3/2})_y$

$((CH_3)SiO_{3/2})_c$ ,

$(SiO_{4/2})_d$ ;

a propyl-phenyl silsesquioxane resin comprising the units:

$(CH_3CH_2CH_2SiO_{3/2})_x$

$(C_6H_5SiO_{3/2})_y$

$((CH_3)_3SiO_{1/2})_a$ ,

$((CH_3)_2SiO_{2/2})_b$ ,

$((CH_3)SiO_{3/2})_c$ , and

$(SiO_{4/2})_d$ ;

a propyl-phenyl silsesquioxane resin comprising the units:

$(CH_3CH_2CH_2SiO_{3/2})_x$

$(C_6H_5SiO_{3/2})_y$

$((CH_3)(H_2NCH_2CH_2CH_2SiO_{2/2})_b$ ; or

a propyl-phenyl silsesquioxane resin comprising the units:

$(CH_3CH_2CH_2SiO_{3/2})_x$

$(C_6H_5SiO_{3/2})_y$

$((CH_3)(C_6H_5)SiO_{2/2})_b$ ;

wherein a, b, c, and d have value of zero to 0.4, x and y have a value of 0.05 to 0.95, with the provisos that the value of x + y is equal to or greater than 0.60, and the value of a + b + c + d + x + y = 1.

**5.** The cosmetic product of any one of claims 1 to 4 further comprising:

(C) a volatile siloxane or organic solvent.

**6.** The cosmetic product of any one of claims 1 to 5 where the cosmetic product is a lipstick or foundation.

**Patentansprüche**

**1.** Ein kosmetisches Produkt, dass eine Zusammensetzung umfasst, enthaltend:

(A) ein Pulver und
(B) ein Alkylphenylsilsesquioxanharz mit wenigstens 60 Mol-%

Siloxyeinheiten mit der Formel $(R'SiO_{3/2})_x(C_6H_5SiO_{3/2})_y$,
worin x und y einen Wert von 0,05 bis 0,95 haben und
R' eine einbindige Kohlenwasserstoffgruppe mit 2 bis 8 Kohlenstoffatomen ist.

**2.** Das kosmetische Produkt gemäß Anspruch 1, wobei das Alkylphenylsilsesquioxanharz die Einheiten umfasst:

(i) $(R^1{}_3SiO_{1/2})_a$
(ii) $(R^2{}_2SiO_{2/2})_b$

(iii) $(R^3SiO_{3/2})_c$
(iv) $(SiO_{4/2})_d$
(v) $(R'SiO_{3/2})_x$ und
(vi) $(C_6H_5SiO_{3/2})_y$ ;

worin
$R^1$, $R^2$ und $R^3$ unabhängig voneinander Alkylgruppen mit 1 bis 8 Kohlenstoffatomen, eine Arylgruppe, eine Carbinolgruppe oder eine Aminogruppe sind,
R' eine einbindige Kohlenwasserstoffgruppe mit 2 bis 8 Kohlenstoffatomen ist,

a, b, c und d einen Wert von null bis 0,4 haben,
x und y einen Wert von 0,05 bis 0,95 haben,
unter den Voraussetzungen, dass der Wert von x + y gleich oder größer als 0,60 ist und der Wert von a + b + c + d + x + y = 1 ist.

**3.** Das kosmetische Produkt gemäß Anspruch 1 oder 2, wobei R' eine Propylgruppe ist.

**4.** Das kosmetische Produkt gemäß einem der Ansprüche 1 bis 3, enthaltend:

ein Propylphenylsilsesquioxanharz, enthaltend die Einheiten:

$(CH_3CH_2CH_2SiO_{3/2})_x$

$(C_6H_5SiO_{3/2})_y$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)_3SiO_{1/2})_a$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)_2SiO_{2/2})_b$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)SiO_{3/2})_c$ ;

ein Propylphenylsilsesquioxanharz, enthaltend die Einheiten:

$(CH_3CH_2CH_2SiO_{3/2})_x$

$(C_6H_5SiO_{3/2})_y$

$(SiO_{4/2})_d$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)_3SiO_{1/2})_a$

$(SiO_{4/2})_d$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)_3SiO_{1/2})_a$

$((CH_3)SiO_{3/2})_c$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)_3SiO_{1/2})_a$

$((CH_3)_2SiO_{2/2})_b$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)_2SiO_{2/2})_b$

$((CH_3)SiO_{3/2})_c$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)_2SiO_{2/2})_b$

$(SiO_{4/2})_d$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)SiO_{3/2})_c$

$(SiO_{4/2})_d$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)_3SiO_{1/2})a$

$((CH_3)_2SiO_{2/2})_b$

$((CH_3)SiO_{3/2})_c$ und

$(SiO_{4/2})_d$ ;

ein Propylphenylsilsesquioxanharz, enthaltend die Einheiten:

$(CH_3CH_2CH_2SiO_{3/2})_x$

$(C_6H_5SiO_{3/2})_y$

$((CH_3)(H_2NCH_2CH_2CH_2SiO_{2/2})_b$ oder

ein Propylphenylsilsesquioxanharz, enthaltend die Einheiten:

$(CH_3CH_2CH_2SiO_{3/2})_x$

$(C_6H_5SiO_{3/2})_y$

$((CH_3)(C_6H_5)SiO_{2/2})_b$ ;

worin a, b, c und d einen Wert von null bis 0,4 haben, x und y einen Wert von 0,05 bis 0,95 haben, unter den Voraussetzungen, dass der Wert von x + y gleich oder größer als 0,60 ist und der Wert von a + b + c + d + x + y = 1 ist.

**5.** Das kosmetische Produkt gemäß einem der Ansprüche 1 bis 4, außerdem enthaltend:

(C) ein flüchtiges Siloxan oder organisches Lösungsmittel.

**6.** Das kosmetische Produkt gemäß einem der Ansprüche 1 bis 5, wobei das Kosmetikprodukt ein Lippenstift oder eine Grundierung ist.

## Revendications

**1.** Produit cosmétique qui comprend une composition comprenant :

(A) une poudre, et
(B) une résine d'alkyl-phényl silsesquioxane comprenant au moins 60 % en mole de motifs siloxy répondant à la formule $(R'SiO_{3/2})x(C_6H_5SiO_{3/2})_y$,

où x et y ont une valeur de 0,05 à 0,95, et
R' est un groupe hydrocarbure monovalent comportant 2 à 8 atomes de carbone.

**2.** Produit cosmétique selon la revendication 1, dans lequel la résine d'alkyl-phényl silsesquioxane comprend les motifs :

(i) $(R^1{}_3SiO_{1/2})a$,
(ii) $(R^2{}_2SiO_{2/2})_b$,

(iii) $(R^3SiO_{3/2})_c$,
(iv) $(SiO_{4/2})_d$,
(v) $(R'SiO_{3/2})_x$ et
(vi) $(C_6H_5SiO_{3/2})_y$,

dans lesquels
$R^1$, $R^2$ et $R^3$ sont indépendamment un groupe alkyle comportant 1 à 8 atomes de carbone, un groupe aryle, un groupe carbanol, ou un groupe amino,
R' est un groupe hydrocarbure monovalent comportant 2 à 8 atomes de carbone,
a, b, c et d ont une valeur de zéro à 0,4,
x et y ont une valeur de 0,05 à 0,95,
à condition que la valeur de x + y soit égale ou supérieure à 0,60 et que lavaleurdea+b+c+d+x+y= 1.

**3.** Produit cosmétique selon la revendication 1 ou la revendication 2, où R' est un groupe propyle.

**4.** Produit cosmétique selon l'une quelconque des revendications 1 à 3, comprenant :

une résine de propyl-phényl silsesquioxane comprenant les motifs :

$(CH_3CH_2CH_2SiO_{3/2})_x$,

$(C_6H_5SiO_{3/2})_y$ ;

une résine de propyl-phényl silsesquioxane comprenant les motifs :

$(CH_3CH_2CH_2SiO_{3/2})_x$,

$(C_6H_5SiO_{3/2})_y$,

$((CH_3)_3SiO_{1/2})_a$ ;

une résine de propyl-phényl silsesquioxane comprenant les motifs :

$(CH_3CH_2CH_2SiO_{3/2})_x$,

$(C_6H_5SiO_{3/2})_y$,

$((CH_3)_2SiO_{2/2})_b$ ;

une résine de propyl-phényl silsesquioxane comprenant les motifs :

$(CH_3CH_2CH_2SiO_{3/2})_x$,

$(C_6HsSiO_{3/2})_y$,

$((CH_3)SiO_{3/2})_c$ ;

une résine de propyl-phényl silsesquioxane comprenant les motifs :

$(CH_3CH_2CH_2SiO_{3/2})_x$,

$(C_6H_5SiO_{3/2})_y$,

$(SiO_{4/2})_d$ ;

une résine de propyl-phényl silsesquioxane comprenant les motifs :

$(CH_3CH_2CH_2SiO_{3/2})_x$,

$(C_6H_5SiO_{3/2})_y$,

$((CH_3)_3SiO_{1/2})_a$,

$(SiO_{4/2})_d$ ;

une résine de propyl-phényl silsesquioxane comprenant les motifs :

$(CH_3CH_2CH_2SiO_{3/2})_x$,

$(C_6H_5SiO_{3/2})_y$,

$((CH_3)_3SiO_{1/2})_a$,

$((CH_3)SiO_{3/2})_c$;

une résine de propyl-phényl silsesquioxane comprenant les motifs :

$(CH_3CH_2CH_2SiO_{3/2})_x$,

$(C_6H_5SiO_{3/2})_y$,

$((CH_3)_3SiO_{1/2})_a$,

$((CH_3)_2SiO_{2/2})_b$;

une résine de propyl-phényl silsesquioxane comprenant les motifs :

$(CH_3CH_2CH_2SiO_{3/2})_x$,

$(C_6H_5SiO_{3/2})_y$,

$((CH_3)_2SiO_{2/2})_b$,

$((CH_3)SiO_{3/2})_c$;

une résine de propyl-phényl silsesquioxane comprenant les motifs :

$(CH_3CH_2CH_2SiO_{3/2})_x$,

$(C_6H_5SiO_{3/2})_y$,

$((CH_3)_2SiO_{2/2})_b$,

$(SiO_{4/2})_d$ ;

une résine de propyl-phényl silsesquioxane comprenant les motifs :

$(CH_3CH_2CH_2SiO_{3/2})_x$,

$(C_6H_5SiO_{3/2})_y$,

$((CH_3)SiO_{3/2})_c$,

$(SiO_{4/2})_d$ ;

une résine de propyl-phényl silsesquioxane comprenant les motifs :

$(CH_3CH_2CH_2SiO_{3/2})_x$,

$(C_6H_5SiO_{3/2})_y$,

$((CH_3)_3SiO_{1/2})_a$,

$((CH_3)_2SiO_{2/2})_b$,

$((CH_3)SiO_{3/2})_c$, et

$(SiO_{4/2})_d$ ;

une résine de propyl-phényl silsesquioxane comprenant les motifs :

$(CH_3CH_2CH_2SiO_{3/2})_x$,

$(C_6H_5SiO_{3/2})_y$,

$((CH_3)(H_2NCH_2CH_2CH_2SiO_{2/2})_b$ ; ou

une résine de propyl-phényl silsesquioxane comprenant les motifs :

$(CH_3CH_2CH_2SiO_{3/2})_x$,

$(C_6H_5SiO_{3/2})_y$,

$((CH_3)(C_6H_5)SiO_{2/2})_b$ ;

dans lesquels a, b, c et d ont une valeur de zéro à 0,4, x et y ont une valeur de 0,05 à 0,95, à condition que la valeur de x + y soit égale ou supérieure à 0,60 et que la valeur de a + b + c + d + x + y = 1.

**5.** Produit cosmétique selon l'une quelconque des revendications 1 à 4, comprenant en outre :

(C) un solvant siloxane ou organique volatil.

**6.** Produit cosmétique selon l'une quelconque des revendications 1 à 5, où le produit cosmétique est un fard à lèvres ou un fond de teint.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6071503 A
- US 6074654 A
- US 6139823 A
- US 6340466 B
- WO 9717058 A
- WO 9717059 A
- US 5330747 A
- US 5800816 A
- US 5837223 A
- US 6036947 A
- GB 2319527 A
- JP 6072085 A